# EUROPEAN PATENT APPLICATION

(11) **EP 0 726 072 A2**
(43) Date of publication of application: **14.08.1996**
(21) Application number: 96101756.3
(22) Date of filing: 07.02.1996
(51) Int. Cl.: A61K 31/415, A61K 45/06

(54) **Composition for the treatment of hypertension and congestive heart failure, containing an angiotensin II antagonist and an endopeptidase inhibitor**

(30) Priority: 08.02.1995 US 386366
(71) Applicant: BRISTOL-MYERS SQUIBB COMPANY, Princeton, NJ 08543-4000 (US)
(72) Inventor: Powell, James R., Washington Crossing, PA 18977 (US); Seymour, Andrea Ann, Lambertville, NJ 08530 (US); Trippodo, Nick C., Newtown, PA 18940 (US)
(74) Representative: Josif, Albert, Dr.-Ing.

(57) **Abstract**

Hypertension and/or congestive heart failure are treated with the combination of the angiotensin II antagonist 2-butyl-6,7,8,9-tetrahydro-3-[[2'-(1H-tetrazol-5-yl)[1,1'-biphenyl]-4-yl]methyl]-1,3-diazaspiro[4.4]nonan-4-one and a selective neutral endopeptidase inhibitor or a dual acting neutral endopeptidase inhibitor.

## Description

Darrow et al. in European Patent Application 498,361 disclose treating hypertension or congestive heart failure with a combination of an angiotensin II antagonist or a renin inhibitor with a neutral endopeptidase inhibitor.

Matsumoto et al., JASN, September 1993, disclose that the combined therapy of an angiotensin II blocker, DUP753, and a neutral endopeptidase inhibitor, candoxatril, may be useful in the treatment of congestive heart failure and renal failure.

Bernhart et al. in United States Patent 5,270,317 disclose a series of N-substituted heterocyclic derivatives which possess angiotensin II antagonist activity. Bernhart et al. disclose that such compounds can be used in the treatment of various cardiovascular complaints, especially hypertension, heart failure, and venous insufficiency, as well as in the treatment of glaucoma, diabetic retinopathy and various complaints of the central nervous system. It is also disclosed that such compound can be used in combination with other active agents such as tranquilizers, beta-blocking compounds, a calcium antagonist, or a diuretic.

Selective neural endopeptidase inhibitors are taught by Delaney et al. in United States Patents 4,722,810 and 5,223,516 and the use of selective neutral endopeptidase inhibitors alone or in combination with angiotensin converting enzyme inhibitors to treat hypertension are disclosed by Delaney et al. U.K. Patent Application 2,207,351 and by Haslanger et al. in United States Patent 4,749,688. The treatment of congestive heart failure by administration of a combination of a selective neutral endopeptidase inhibitor and an angiotensin converting enzyme inhibitor is disclosed by Seymour in United States Patent 5,225,401.

Compounds possessing both neutral endopeptidase and angiotensin converting enzyme inhibition activity are disclosed by Flynn et al. in United States Patent 5,366,973, European Patent Application 481,522 and PCT Patent Applications WO 93/16103, and WO 94/10193, Warshawsky et al. European Patent Applications 534,363, 534,396 and 534,492, Fournie-Zaluski European Patent Application 524,553, Karanewsky et al. European Patent Application 599,444, Karanewsky European Patent Application 595,610, Robl et al., European Patent Application 629,627, Robl United States Patent 5,362,727 and European Patent Application 657,453.

This invention is directed to the discovery that the angiotensin II antagonist 2-butyl-6,7,8,9-tetrahydro-3-[[2'-(1H-tetrazol-5-yl)[1,1'-biphenyl]-4 -yl]methyl]-1,3-diazaspiro[4.4]nonan-4-one acts synergistically with a selective neutral endopeptidase inhibitor or a dual acting neutral endopeptidase inhibitor as defined below to reduce cardiac preload and afterload and enhance natriureses. The combination of this angiotensin II antagonist and the selective or dual acting neutral endopeptidase inhibitor produced significant reductions in left ventricular end diastolic pressure (LVEDP) and left ventricular systolic pressure (LVSP) that were greater than those produced by either treatment alone. Thus, the combination of this particular angiotensin II antagonist and the selective or dual acting neutral endopeptidase inhibitor is useful in treating hypertension and/or congestive heart failure.

The angiotensin II antagonist employed within this invention is the compound 2-butyl-6,7,8,9-tetrahydro-3-[[2'-(1H-tetrazol-5-yl)[1,1'-biphenyl]-4 -yl]methyl]-1,3-diazaspiro[4.4]nonan-4-one having the structural formula 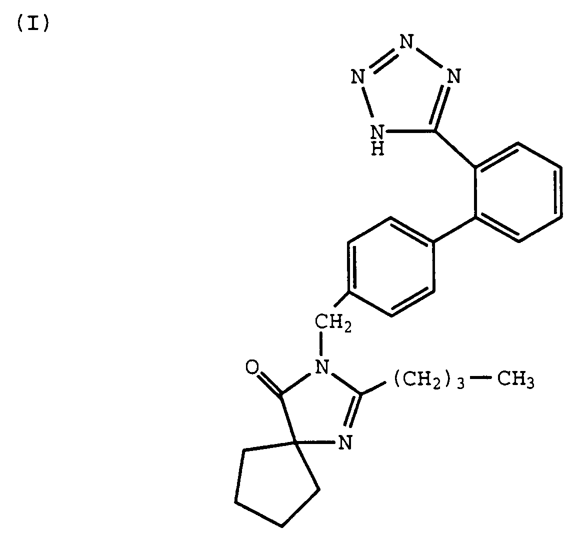 known in the literature as SR47436, BMS 186295, or irbesartan and pharmaceutically acceptable salts thereof such as the potassium and sodium salts. These angiotensin II antagonists and their method of preparation are disclosed by Bernhart et al. in United States Patent 5,270,317.

The selective neutral endopeptiadase inhibitor for use within this invention are those of the formula 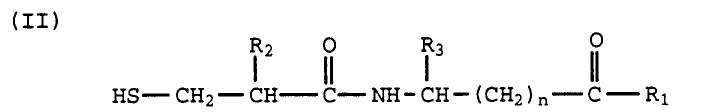 and pharmaceutically acceptable salts thereof wherein:
R₂ is alkyl of 1 to 7 carbons, trifluoromethyl, phenyl, substituted phenyl, -(CH₂)_{1 to 4}-phenyl, or -(CH₂)_{1 to 4}-substituted phenyl;
R₃ is hydrogen, alkyl of 1 to 7 carbons, phenyl, substituted phenyl, -(CH₂)_{1 to 4}-phenyl, or -(CH₂)_{1 to 4}-substituted phenyl;
R₁ is hydroxy, alkoxy of 1 to 7 carbons, or NH₂;
n is an integer from 1 to 15; and
the term substituted phenyl refers to a substituent selected from lower alkyl of 1 to 4 carbons, lower alkoxy of 1 to 4 carbons, lower alkylthio of 1 to 4 carbons, hydroxy, Cl, Br, or F.

Preferred are the selective neutral endopeptidase inhibitors of formula II wherein:
R₂ is benzyl;
R₃ is hydrogen;
n is an integer from 1 to 9; and
R₁ is hydroxy.

Most preferred for use in this invention is the selective neutral endopeptidase inhibitor of formula II reported in the literature as SQ 28,603 which is the compound of formula II wherein:
R₂ is benzyl;
R₃ is hydrogen;
n is one; and
R₁ is hydroxy.

The preparation of the selective neutral endopeptidase inhibitors of formula II wherein R₂ is other than trifluoromethyl are disclosed by Delaney et al. in United States Patent 4,722,810. The preparation of the selective neutral endopeptidase inhibitors of formula II wherein R₂ is trifluoromethyl are disclosed by Delaney et al in United States Patent 5,223,516.

Dual acting neutral endopeptidase inhibitors suitable for use within this invention are compounds which possess both neutral endopeptidase inhibiting activity and angiotensin converting enzyme inhibiting activity. Particularly useful are the dual acting inhibitors of the formula 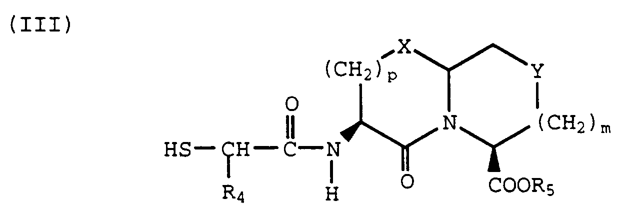 and pharmaceutically acceptable salts thereof wherein:
p is one or two;
X is O or S;
m is zero or one;
Y is CH₂, S or O provided that Y is S or O only when m is one;
R₄ is hydrogen, alkyl of 1 to 7 carbons, phenyl, substituted phenyl, -(CH₂)_{1 to 4}-phenyl, -(CH₂)_{1 to 4}-substituted phenyl, cycloalkyl of 3 to 7 carbons, -(CH₂)_{1 to 4}-cycloalkyl of 3 to 7 carbons, heteroaryl, and -(CH₂)_{1 to 4}-heteroaryl;
R₅ is hydrogen, alkyl of 1 to 7 carbons, -(CH₂)_{1 to 4}-phenyl and -(CH₂)_{1 to 4}-substituted phenyl;
the term substituted phenyl refers to a substituent selected from lower alkyl of 1 to 4 carbons, lower alkoxy of 1 to 4 carbons, lower alkylthio of 1 to 4 carbons, hydroxy, Cl, Br, or F; and
the term heteroaryl refers to monocyclic rings of 5 or 6 atoms containing one or two O and S atoms and/or one to four N atoms provided that the total number of heteroatoms in the ring is 4 or less and bicyclic rings wherein the 5 or 6 membered heteroaryl ring as defined above is fused to a benzene or pyridyl ring.

Preferred are the dual acting neutral endopeptidase inhibitors of formula III wherein:
R₄ is benzyl, cyclopropylmethyl, or straight or branched chain alkyl of 3 to 5 carbons;
p is one or two;
X is O or S;
m is zero or one;
Y is CH₂, S, or O provided that Y is S or O when m is one; and
R₅ is hydrogen.

Most preferred for use in this invention is the dual acting neutral endopeptidase inhibitor of formula III wherein:
R₄ is benzyl;
p is two;
Y is S;
m is one;
Y is CH₂ ; and
R₅ is hydrogen.

The dual acting neutral endopeptidase inhibitors of formula III are disclosed in European Patent Application 629,627 of Robl et al.

Also useful as neutral endopeptidase inhibitors for use within this invention are the dual acting inhibitors of the formula 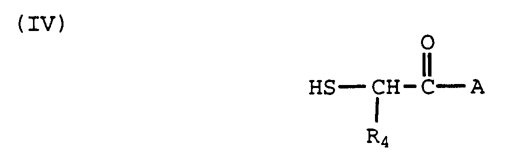 and pharmaceutically acceptable salts thereof wherein:
- A: is 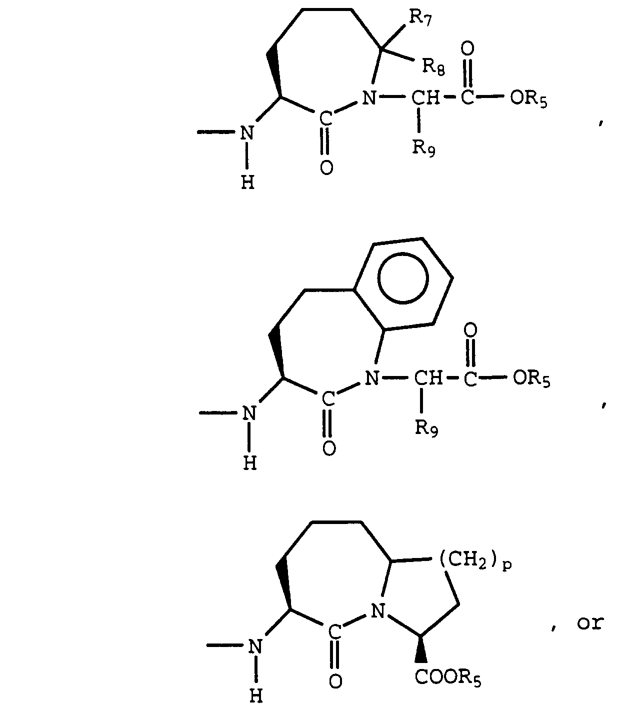 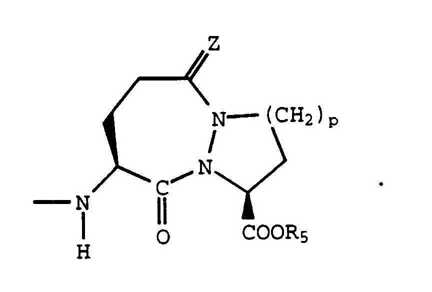 R₄, R₅, and p are as defined above;
R₇ and R₈ are both hydrogen, or both alkyl of 1 to 7 carbons, or R₇ is hydrogen and R₈ is alkyl of 1 to 7 carbons, phenyl, -(CH₂)_{1 to 4}-phenyl and -(CH₂)_{1 to 4}-substituted phenyl, or R₇ and R₈ taken together with the carbon to which they are attached complete a cycloalkyl of 3 to 5 carbons.
R₉ is hydrogen or alkyl of 1 to 7 carbons.
Z is oxo or two hydrogens.

Preferred are the dual acting neutral endopeptidase inhibitors of formula IV wherein:
R₄ is benzyl;
R₇ and R₈ are both methyl;
R₉ is hydrogen or methyl, especially hydrogen;
p is one or two; and
Z is oxo.

The compounds of formula IV and their method of preparation are disclosed in European Patent Application 599,444 and U.S. Patent Application Serial No. 160,540 filed December 1, 1993.

The angiotensin II antagonist 2-butyl-6,7,8,9-tetrahydro-3-[[2'-(1H-tetrazol-5-yl)[1,1'-biphenyl]-4-yl]methyl-1,3-diazaspiro[4.4]nonan-4-one and the selective neutral endopeptidase inhibitor or dual acting neutral endopeptidase inhibitor may be administered from a single dosage form containing both types of compounds, may be administered in separate dosage forms taken at the same time, or may be administered separately on a carefully coordinated schedule. If administered separately, the two compounds can be administered from within several minutes of each other up to about 4 hours apart.

The selective or dual acting neutral endopeptidase inhibitor can be administered at a dosage range of from about 0.03 to about 1000 mg. per kg. of body weight per day with a dosage range of from about 0.3 to about 300 mg. per kg. of body weight per day being preferred. The angiotensin II antagonist can be administered at a dosage range of from about 0.001 to about 50 mg. per kg. of body weight with a dosage range of from about 0.1 to about 10 mg. per kg. of body weight being preferred.

Both compounds can be administered orally, parenterally, or one orally and the other parenterally. Each compound may be administered from one to about four times per day depending upon the duration of activity of the compounds and the severity of the congestive heart failure and/or hypertension being treated.

The compounds can be formulated, in the amounts described above, according to accepted pharmaceutical practice with a physiologically acceptable vehicle, carrier, excipient, binder, preservative, stabilizer, flavor, etc., in the particular type of unit dosage form.

Illustrative of the adjuvents which may be incorporated in tablets are the following: a binder such as gum tragacanth, acacia, corn starch or gelatin; an excipient such as dicalcium phosphate or cellulose; a disintegrating agent such as corn starch, potato starch, alginic acid of the like; a lubricant such as stearic acid or magnesium stearate; a sweetening agent such as sucrose, aspartame, lactose or saccharin; a flavoring agent such as orange, peppermint, oil of wintergreen or cherry. When the dosage unit form is a capsule, it may contain in addition to materials of the above type a liquid carrier such as a fatty oil. Various other materials may be present as coatings or to otherwise modify the physical form of the dosage unit. For instance, tablets or capsules may be coated with shellac, sugar or both. A syrup of elixir may contain the active compound, water, alcohol or the like as the carrier, glycerol as stabilizer, sucrose as sweetening agent, methyl and propyl parabens as preservatives, a dye and a flavoring such as cherry or orange.

In the following examples, BMS 186295 refers to SR47436, i.e. the compound 2-butyl-6,7,8,9-tetrahydro-3-[[2'-(1H-tetrazol-5-yl)[1,1'-biphenyl]-4-yl]-methyl]-1,3-diazaspiro[4.4]nonan-4-one, and SQ 28603 refers to the compound (±)-N-[2-(mercaptomethyl)-1-oxo-3-phenylpropyl]-β-alanine.

### Example 1

The studies described in this experiment were conducted in male hamsters of the BIO TO-2 strain when they were approximately 260 days of age and weighed on average 115 g. These animals develop a genetic form of cardiomyopathy that progresses uniformly among animals through different stages of heart failure. By 240 - 300 days of age the cardiomyopathic hamsters are characterized (as compared with control hamsters) by low mean arterial pressure, a 40% reduction in cardiac output and a decrease in renal blood flow. They display elevated cardiac filling pressure, depressed ventricular function, increased peripheral vascular resistance and have an 8-10-fold increase in plasma atrial natriuretic peptide concentration. Since most animals at this age do not have gross peripheral edema or elevated plasma renin activity, the cardiomyopathic hamsters were considered to be in compensated heart failure.

The experiments were conducted in conscious, unrestrained, cardiomyphathic hamsters three hours after placement of cardivascular catheters using brief anesthesia. The catheters allowed measurement of mean arterial pressure, left ventricular end diastolic pressure, left ventricular systolic pressure and heart rate, and provided a means for the administration of agents intravenously.

### a) Inhibition of The Pressor Response To Angiotensin II

Preliminary experiments were conducted in conscious cardiomyopathic hamsters to determine a dose regimen of BMS 186295 that would nearly completely block the pressor response to angiotensin II for at least two hours. The pressor responses to two challenges of angiotensin II (100 ng/kg, i.v. dissolved in 0.9% sodium chloride, 1 ml/kg) were determined. This dose of angiotensin II produced over a 30% increase in mean arterial pressure. Based on the preliminary experiments, BMS 186295 was administered to 5 cardiomyopathic hamsters at 30 µmol/kg, i.v. followed by continuous i.v. infusion at 1 µmol/kg per min. Challenges of angiotensin II were then repeated at 10- to 30-minute intervals up to 150 minutes following the bolus injection of BMS 186295. The results are shown below.

| Minutes | Change in mean arterial pressure, mm Hg |
|---|---|
| -20′ | 29±2 |
| -10′ | 31±3 |
| BMS-186295, | 30 µmol/kg, i.v. followed by 1 µmol/kg/min, i.v. |
| 10′ | 3±2 |
| 20′ | 2±1 |
| 30′ | 3±2 |
| 40′ | 2±1 |
| 50′ | 1±1 |
| 60′ | 4±1 |
| 70′ | 5±2 |
| 80′ | 4±1 |
| 90′′ | 3±1 |
| 120 | 3±1 |
| 150′ | 2±1 |

### b) Cardiovascular Effects Of BMS 186295, SQ 28603, And the Combination Of These Agents

In this series of experiments baseline measurements of left ventricular end diastolic pressure, left ventricular systolic pressure and heart rate were determined in groups of conscious cardiomyopathic hamsters. Compounds or vehicle were administered intravenously, and measurements were repeated at 30-minute intervals up to 90 minutes after administration of the last agent. BMS 186295 was administered at 30 µmol/kg, i.v. (0.3 ml) followed by a continuous i.v. infusion at 1 µmol/kg per min (0.01 ml/min). BMS 186295 was prepared in 0.028 M potassium hydroxide and diluted to a final concentration of 0.17 M potassium hydroxide. Potassium hydroxide solution (0.17 M) was administered intravenously to the vehicle group at 0.3 ml followed by a continuous infusion at 0.01 ml/min. SQ 28603 was dissolved in 0.84% sodium bicarbonate and administered at 30 µmol/kg, i.v. This dose of SQ 28603 was previously shown to result in a doubling of plasma atrial natriuretic peptide concentration within 90 minutes in this model. One group of cardiomyopathic hamsters received the combination of BMS 186295 and SQ 28603. In this group BMS 186295 was administered according to the same dosage regimen described above; 30 minutes after the bolus injection of BMS 186295, SQ 28603 was administered at 30 µmol/kg, i.v.

Differences in age, body weight and baseline values among groups were evaluated by analysis of variance. Differences in changes from baseline among groups were evaluated by analysis of covariance with repeated measures and contrasts. The baseline value for each variable was used as the covariate. The level of significance was taken at P < 0.05. All data are expressed as mean ± standard error of the mean.

| Left Ventricular End Diastolic Pressure (mm Hg) | | | | |
|---|---|---|---|---|
| Minutes | Vehicle | SQ 28603 | BMS 186295 | BMS 186295 & SQ 28603 |
| Baseline | 19 ± 2 | 18 ± 3 | 17 ± 2 | 21 ± 2 |
| BMS 186295 SQ 28603 | 18 ± 2 | 14 ± 3 | 18 ± 2 | 20 ± 2 |
| 30′ | 19 ± 1 | 14 ± 2 | 16 ± 2 | 12 ± 1 |
| 60′ | 18 ± 1 | 17 ± 3 | 16 ± 2 | 11 ± 2 |
| 90′ | 16 ± 2 | 16 ± 3 | 18 ± 3 | 10 ± 1 |

| Change From Baseline (mm Hg) | | | | |
|---|---|---|---|---|
| Minutes after last treatment | Vehicle | SQ 28603 | BMS 186295 | BMS 186296 & SQ 28603 |
| 30 | 1 ± 1 | 4 ± 1* | -1 ± 1 | -10 ± 2* |
| 60 | -1 ± 1 | -1 ± 2 | -1 ± 1 | -10 ± 3*† |
| 90 | -3 ± 1 | -2 ± 1 | 1 ± 2 | -11 ± 3*† |

| | | | | |
|---|---|---|---|---|
| *P <0.05 vs Vehicle | | | | |
| †P <0.05 vs SQ 28603 | | | | |

| Left Ventricular Systolic Pressure (mm Hg) | | | | |
|---|---|---|---|---|
| Minutes | Vehicle | SQ 28603 | BMS 186295 | BMS 186295 & SQ 28603 |
| Baseline | 111 ± 3 | 117 ± 5 | 112 ± 2 | 107 ± 3 |
| BMS 186295 SQ 28603 | 111 ± 3 | 108 ± 5 | 111 ± 1 | 104 ± 4 |
| 30′ | 111 ± 5 | 109 ± 5 | 114 ± 2 | 92 ± 2 |
| 60′ | 108 ± 3 | 105 ± 4 | 111 ± 2 | 88 ± 5 |
| 90′ | 105 ± 5 | 105 ± 5 | 112 ± 5 | 89 ± 4 |

| Change From Baseline (mm Hg) | | | | |
|---|---|---|---|---|
| Minutes After Last Treatment | Vehicle | SQ 28603 | BMS 186295 | BMS 186296 & SQ 28603 |
| 30 | -1 ± 3 | -8 ± 1* | 2 ± 2 | -16 ± 3*† |
| 60 | -3 ± 1 | -12 ± 3* | -1 ± 2 | -20 ± 4*† |
| 90 | -6 ± 4 | -12 ± 2 | 1 ± 5 | -18 ± 4* |

| | | | | |
|---|---|---|---|---|
| *P <0.05 vs Vehicle | | | | |
| †P <0.05 vs SQ 28603 | | | | |

| Heart Rate (beats/min) | | | | |
|---|---|---|---|---|
| Minutes | Vehicle | SQ 28603 | BMS 186295 | BMS 186295 & SQ 28603 |
| Baseline | 350 ± 10 | 378 ± 12 | 338 ± 16 | 364 ± 6 |
| BMS 186295 SQ 28603 | 365 ± 8 | 380 ± 7 | 364 ± 14 | 366 ± 12 |
| 30′ | 347 ± 15 | 381 ± 9 | 363 ± 20 | 366 ± 11 |
| 60′ | 345 ± 15 | 366 ± 9 | 367 ± 18 | 353 ± 10 |
| 90′ | 354 ± 13 | 378 ± 8 | 369 ± 28 | 351 ± 9 |

| Change From Baseline (mm Hg) | | | | |
|---|---|---|---|---|
| Minutes after last treatment | Vehicle | SQ 28603 | BMS 186295 | BMS 186296 & SQ 28603 |
| 30 | -3 ± 15 | 3 ± 8 | 25 ± 9 | 1 ± 6 |
| 60 | -5 ± 15 | -13 ± 12* | 29 ± 7* | -11 ± 9 |
| 90 | 7 ± 13 | -1 ± 9 | 44 ± 11* | -14 ± 5 |

| | | | | |
|---|---|---|---|---|
| *P <0.05 vs Vehicle | | | | |

### Discussion of Results

Following the administration of BMS 186295, the pressor responses to angiotensin II were less than 17% of the response before the administration of the inhibitor. These results indicate that nearly complete inhibition of the pressor response to angiotensin II was achieved following the administered dosage regimen of BMS 186295, and suggests effective blockade of the angiotensin II receptors.

The combination of BMS 186295 and SQ 28603 produced cardiovascular effects that were greater than those with either treatment alone. Specifically, the combination caused significant decreases in left ventricular end diastolic pressure and left ventricular systolic pressure with no significant change in heart rate. SQ 28603 produced smaller decreases, whereas BMS 186295 had no significant effects on the measured cardiovascular pressures. Thus, the combination of BMS 186295 and SQ 28603 produced beneficial hemodynamic effects in cardiomyopathic hamsters with compensated heart failure.

### Example 2

The studies described in this experiment were conducted in dogs that had been rendered hypertensive by prior unilateral nephrectomy and constriction of the remaining renal artery. This model is characterized by normal basal levels of plasma renin activity and is relatively resistant to the anti-hypertensive activity of angiotensin converting enzyme inhibitors and AT₁ receptor antagonists. Furthermore, the 1-kidney-1-clip (IKIC) hypertensive dogs have normal plasma concentrations of atrial natriutetic peptide and fail to develop depressor responses to neutral endopeptidase inhibitors.

The following experiments were conducted in fasted 1K1C hypertensive dogs lightly restrained in standard canine slings. An indwelling arterial catheter was accessed via a subcutaneous port for measurement of blood pressure via a Gould-Statham pressure transducer. Mean arterial pressure (MAP) was continuously recorded on a Gould chart writer and stored electronically using a Po-Ne-Mah data acquisition system. During each study, urine was collected at 20 minute intervals via a Foley bladder catheter for determination of urine volume. The concentrations of urinary sodium and potassium were measured using ion-selective electrodes and their rates of urinary excretion (µEq/min) were calculated. Glomerular filtration rate (GFR) and effective renal plasma flow (ERPF) were determined by the renal clearances of exogenous creatinine and para-aminohippuric acid (PAH), respectively. The concentrations of creatinine and PAH in sequential samples of urine and plasma were determined by spectrophotometric assays and the clearances were calculated by the standard formula.

Arterial blood samples were drawn at the end of the control period and at 60 minute intervals thereafter for determination of the plasma concentrations of atrial natriuretic peptide (ANP), cyclic GMP and plasma renin activity (PRA) by separate radioimmunoassays. The plasma and urine samples were preserved and the assays were conducted according to standard radioimmunoassay procedures. Urinary excretion rates of cyclic GMP and ANP were calculated and expressed as pmol/min and fmol/min, respectively.

Four 1K1C hypertensive dogs were treated with the combination of 30 µmol/kg iv of BMS 186295 and 30 µmol/kg iv of SQ 28603. Vehicle (0.84% sodium bicarbonate), 30 µmol/kg iv of SQ 28603 and 30 µmol/kg iv of BMS 186295 were tested in 3 additional groups of 1K1C hypertensive dogs (n=4 to 5/treatment). In each study, baseline measurements were obtained during two 20 minute control periods. One of the treatments was then administered and sampling continued at 20 minute intervals for three hours.

To minimize inter-animal variability, each data point was expressed as the change from the average control value for that parameter. Significant differences among treatments were identified by analysis of variance for repeated measures. Contrasts were calculated to identify significant differences from the effects of vehicle and to compare the combination of SQ 28603 and BMS 186295 to the individual treatments. Results are given as mean ± SEM.

### Results

**Table 1**

| Mean Arterial Pressure (mm Hg) | | | | |
|---|---|---|---|---|
| Time (min) after Treatment | Vehicle (n=5) | SQ 28603 (n=4) | BMS 186295 (n=4) | SQ 28603 + BMS 186295 (n=4) |
| Control | 132±3 | 132±8 | 157±7 | 140±3 |
| | Change from control | | | |
| 20 | 3±1 | 6±1 | -1±1 | 2±2 |
| 40 | 3±2 | 9±3 * | -10±1 * | 2±1 †§ |
| 60 | 2±1 | 8±3 * | -8±2 * | 0±2 †§ |
| 80 | 6±2 | 8±2 | -5±2 * | -1±3 * |
| 100 | 6±2 | 5±3 | -4±1* | 1±2 † |
| 120 | 6±2 | 3±4 | -1±2 * | 2±2 |
| 140 | 7±2 | 4±2 | -1±2 * | 6±2 † |
| 160 | 5±2 | 5±2 | 0±2 | 9±3 † |
| 180 | 6±3 | 7±3 | -1±3 * | 6±4 † |

| | | | | |
|---|---|---|---|---|
| * p<0.05 compared to vehicle | | | | |
| † p<0.05 compared to BMS 186295 | | | | |
| § p<0.05 compared to SQ 28603 | | | | |

BMS 186295 signigicantly reduced mean arterial pressure (MAP) (Table 1) in the conscious 1K1C hypertensive dogs whereas SQ 28603 initially increased MAP. The effects of the combination BMS 186295 and SQ 28603 were not consistently different from those of vehicle.

**TABLE 2**

| Sodium Excretion (µEq/min) | | | | |
|---|---|---|---|---|
| Time (min) after Treatment | Vehicle (n=5) | SQ 28603 (n=4) | BMS 186295 (n=4) | SQ 28603 + BMS 186295 (n=4) |
| Control | 60±14 | 40±20 | 18±2 | 18±6 |
| | Change from control | | | |
| 20 | -17±10 | 13±4 | 23±9 | 62±31 *†§ |
| 40 | -16±12 | 21±16 * | 41±13 * | 83±34 *†§ |
| 60 | -12±14 | 14±9 * | 36±8 * | 87±18 *†§ |
| 80 | -11±14 | 25±13 * | 27±6 * | 70±16 *†§ |
| 100 | -12±11 | 24±12 * | 22±3 * | 54±12 *†§ |
| 120 | -16±13 | 35±19 * | 30±4 * | 60±21 *†§ |
| 140 | -10±14 | 39±18 * | 28±7 * | 69±22 *†§ |
| 160 | -4±15 | 44±19 * | 30±7 * | 68±16 *† |
| 180 | -3±13 | 44±18 * | 30±2 * | 74±19 *†§ |

| | | | | |
|---|---|---|---|---|
| * p<0.05 compared to vehicle | | | | |
| *† p<0.05 compared to *vehicle or †BMS186295 | | | | |
| *†§ p<0.05 compared to *vehicle, †BMS186295 or SQ28603 | | | | |

**TABLE 3**

| Urine Volume (ml/min) | | | | |
|---|---|---|---|---|
| Time (min) after Treatment | Vehicle (n=5) | SQ 28603 (n=4) | BMS 186295 (n=4) | SQ 28603 + BMS 186295 (n=4) |
| Control | 0.64±0.14 | 0.38±0.13 | 0.43±0.16 | 0.36±0.13 |
| | Change from control | | | |
| 20 | -0.20±0.12 | 0.29±0.31 * | 0.01±0.07 | 0.32±0.17 * |
| 40 | -0.27±0.14 | 0.15±0.13 * | 0.09±0.14 * | 0.51±0.22 *†§ |
| 60 | -0.26±0.14 | 0.18±0.14 * | 0.12±0.07 * | 0.51±0.11 *†§ |
| 80 | -0.26±0.15 | 0.21±0.15 * | 0.05±0.06 * | 0.34±0.08 *† |
| 100 | -0.23±0.14 | 0.07±0.08 * | -0.02±0.09 * | 0.14±0.08 * |
| 120 | -0.30±0.13 | 0.12±0.13 * | -0.02±0.10 * | 0.20±0.07 * |
| 140 | -0.25±0.14 | 0.20±0.10 * | -0.07±0.12 * | 0.26±0.09 *† |
| 160 | -0.23±0.15 | 0.24±0.11 * | -0.07±0.16 | 0.16±0.03 *† |
| 180 | -0.22±0.14 | 0.17±0.06 * | -0.02±0.07 * | 0.17±0.03 * |

| | | | | |
|---|---|---|---|---|
| * p<0.05 compared to vehicle | | | | |
| *† p<0.05 compared to *vehicle or †BMS186295 | | | | |
| *†§ p<0.05 compared to *vehicle, †BMS186295 or SQ28603 | | | | |

BMS 186295 and SQ 28603 each individually increased sodium excretion (TABLE 2) and urine volume (TABLE 3) in the conscious 1K1C hypertensive dogs. The natriuretic response to the combination of BMS 186295 and SQ 28603 was greater than the activity of either of the compounds administered singly. The increase in the amount of sodium excreted during the 3 hours after simultaneous injections of BMS 186295 and SQ 28603 (12.6±3.4 mEq/3 hr) approximated the sum of the natriuretic responses to BMS 186295 (5.4±1.0 mEq/3 hr) and to SQ 28603 (5.2±2.3 mEq/3 hr) given individually.

**TABLE 4**

| Glomerular Filtration Rate (ml/min) | | | | |
|---|---|---|---|---|
| Time (min) after Treatment | Vehicle (n=5) | SQ 28603 (n=4) | BMS 186295 (n=4) | SQ 28603 + BMS 186295 (n=4) |
| Control | 50±4 | 39±5 | 45±10 | 46±4 |
| | Change from control | | | |
| 20 | -2±2 | 6±11 | -4±2 | -8±7 § |
| 40 | 0±4 | 3±2 | -2±5 | 6±3 † |
| 60 | 2±3 | 6±5 | 6±0 | 11±2 * |
| 80 | 0±3 | 5±2 | 5±2 | 7±1 * |
| 100 | 4±3 | 5±4 | 3±4 | 4±4 |
| 120 | -1±2 | 5±1 | 4±3 | 12±2 *§ |
| 140 | 1±2 | 7±4 | 0±5 | 9±1 * |
| 160 | 5±4 | 9±5 | 0±7 | 6±2 |
| 180 | 5±3 | 10±4 | 0±4 | 5±5 |

| | | | | |
|---|---|---|---|---|
| * p<0.05 compared to vehicle | | | | |
| § p<0.05 compared to SQ 28603 | | | | |
| † p<0.05 compared to BMS 186295 | | | | |
| *§ p<0.05 compared to *vehicle or §SQ28603 | | | | |

**TABLE 5**

| Effective Renal Plasma Flow (ml/min) | | | | |
|---|---|---|---|---|
| Time (min) after Treatment | Vehicle (n=5) | SQ 28603 (n=4) | BMS 186295 (n=3) | SQ 28603 + BMS 186295 (n=4) |
| Control | 144±18 | 123±15 | 127±41 | 142±30 |
| | Change from control | | | |
| 20 | -25±10 | 6±38 | -68±30 * | -84±19 *§ |
| 40 | -27±17 | -5±15 | -54±35 | -64±27 *§ |
| 60 | -25±16 | 3±20 | -35±24 | -51±21 § |
| 80 | 30±18 | -20±6 | -13±9 | -45±21 † |
| 100 | -26±14 | -25±10 | -1±10 | -41±23 † |
| 120 | -41±14 | -15±6 | -8±17 * | -15±8 |
| 140 | -32±12 | -11±12 | 8±3 * | -12±12 |
| 160 | -21±13 | -1±12 | -14±12 | -28±14 |
| 180 | -21±15 | -4±9 | -12±11 | -23±16 |

| | | | | |
|---|---|---|---|---|
| * p<0.05 compared to vehicle | | | | |
| *§ p<0.05 compared to *vehicle or §SQ28603 | | | | |
| † p<0.05 compared to BMS 186295 | | | | |
| § p<0.05 compared to SQ 28603 | | | | |

The combination of BMS 186295 and SQ 28603 significantly increased GFR (TABLE 4) at several times during the 3 hour test when compared with the effects of vehicle even though effective renal plasma flow (TABLE 5) did not increase. The increase in GFR alone did not account for the full natriuretic response, as indicated by a significantly rise in fractional sodium excretion from 0.26±0.07% to 1.28±0.29%.

**TABLE 6**

| ANP Excretion (fmol/min) | | | | |
|---|---|---|---|---|
| Time (min) after Treatment | Vehicle (n=5) | SQ 28603 (n=4) | BMS 186295 (n=4) | SQ 28603 + BMS 186295 (n=4) |
| Control | 1.2±0.1 | 1.9±0.2 | 1.5±0.6 | 1.5±0.4 |
| | Change from control | | | |
| 20 | -0.1±0.1 | 20.8±4.9 * | -0.7±0.5 | 3.7±1.5 |
| 40 | -0.1±0.1 | 24.0±4.6 * | -0.6±0.5 | 13.8±2.2 |
| 60 | -0.1±0.1 | 45.2±24.2 * | -0.2±0.3 | 40.7±21.1 *† |
| 80 | -0.1±0.1 | 55.5±19.7 * | -0.4±0.3 | 30.3±8.1 *†§ |
| 100 | -0.0±0.1 | 41.0±12.3 * | -0.6±0.4 | 27.0±10.9 *† |
| 120 | -0.2±0.1 | 48.3±21.5 * | -0.5±0.4 | 39.4±13.7 *† |
| 140 | -0.0±0.1 | 41.8±14.2 * | -0.6±0.4 | 37.2±14.9 *† |
| 160 | 0.3±0.2 | 36.9±12.4 * | -0.5±0.4 | 30.1±12.4 *† |
| 180 | 0.0±0.2 | 29.0±7.9 * | -0.6±0.4 | 33.2±10.0 *†§ |

| | | | | |
|---|---|---|---|---|
| * p<0.05 compared to vehicle | | | | |
| *† p<0.05 compared to *vehicle or †BMS186295 | | | | |
| *†§ p<0.05 compared to *vehicle, †BMS186295 or SQ28603 | | | | |

**TABLE 7**

| Cyclic GMP Excretion (pmol/min) | | | | |
|---|---|---|---|---|
| Time (min) after Treatment | Vehicle (n=5) | SQ 28603 (n=4) | BMS 186295 (n=4) | SQ 28603 + BMS 186295 (n=4) |
| Control | 1106±85 | 1017±180 | 1122±389 | 1030±170 |
| | Change from control | | | |
| 20 | -177±172 | 106±295 | -372±240 | -202±144 |
| 40 | -185±41 | 101±280 | -277±255 | 164±94 |
| 60 | -78±207 | 338±206 | -150±203 | 432±174 |
| 80 | -205±231 | 226±154 | -309±199 | 313±144 |
| 100 | -180±129 | 128±178 | -266±193 | 106±97 |
| 120 | -229±121 | 117±220 | -283±236 | 139±66 |
| 140 | -52±195 | 121±182 | -449±206 | 194±156 |
| 160 | -24±223 | 100±246 | -391±199 | 229±62 |
| 180 | 76±246 | 343±170 | -611±346 | 316±59 |

Urinary excretion of ANP (TABLE 6) increased significantly after administration of SQ 28603 alone and in combination with BMS 186295, indicating that the NEP inhibitor had prevented the degradation of ANP. Cyclic GMP (TABLE 7), the second messenger of the biological ANP receptor, tended to increase in the dogs receiving SQ 28603 alone (+32±28 nmol/3 hr) or the combination of BMS 186295 and SQ 28603 (+34±12 nmol/3 hr), but because of the variability of the response, these changes did not achieve statistical significance compared to vehicle (-21±15 nmol/3 hr). These data suggested that the protection of renal ANP contributed to the natriuretic response. BMS 186295 given alone did not affect ANP excretion nor did it alter the ANP response to SQ 28603. Therefore, the enhanced response to the combination of BMS 186295 and SQ 28603 could not be attributed to an additional effect of the angiotensin II antagonist on the renal metabolism of ANP or the resultant accumulation of cyclic GMP.

**TABLE 8**

| Plasma Renin Activity (pmol AI/ml/hr) | | | | |
|---|---|---|---|---|
| Time (min) after Treatment | Vehicle (n=5) | SQ 28603 (n=4) | BMS 186295 (n=4) | SQ 28603 + BMS 186295 (n=4) |
| Control | 0.45±0.10 | 0.16±0.02 | 0.55±0.07 | -/90±0.09 |
| | Change from control | | | |
| 60 | -0.09±0.07 | -0.07±0.05 | 1.28±0.51 * | 0.39±0.42 † |
| 120 | -0.04±0.11 | -0.02±0.05 | 1.49±0.57 * | 0.58±0.44 *† |
| 180 | -0.03±0.11 | -0.01±0.7 | 1.24±0.62 * | 0.36±0.16 † |

| | | | | |
|---|---|---|---|---|
| * p<0.05 compared to vehicle | | | | |
| † p<0.05 compared to BMS 186295 | | | | |
| *† p<0.05 compared to *vehicle or †BMS186295 | | | | |

Finally, BMS 186295 significantly increased PRA (TABLE 8) indicating that the angiotensin receptor antagonist interrupted the negative feedback of angiotensin II on renin release. The smaller PRA response to BMS 186295 in the presence of SQ 28603 may be attributed to the inhibition of renin release by the increased ANP levels. Alternatively, BMS 186295 may have also activated the intrarenal baroreceptor by virtue of its depressor activity and thereby increased renin secretion.

## Claims

1. Use of angiotensin II antagonist 2-butyl-6,7,8,9-tetrahydro-3-[[2'-(1H-tetrazol-5-yl)-[1,1'-biphenyl]-4-yl]methyl]-1,3-diazaspiro[4.4]-nonan-4-one or a pharmaceutically acceptable salt thereof and a selective neutral endopeptidase inhibitor or a dual acting neutral endopeptidase inhibitor for manufacturing a medicament for treating hypertension and/or congestive heart failure in a mammalian specie in need of such treatment.

2. The use of Claim 1 wherein said endopeptidase inhibitor is a selective neutral endopeptidase inhibitor of the formula 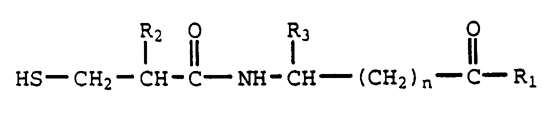 or a pharmaceutically acceptable salt thereof wherein:
R₂ is alkyl of 1 to 7 carbons, trifluoromethyl, phenyl, substituted phenyl, -(CH₂)_{1 to 4}-phenyl or -(CH₂)_{1 to 4}-substituted phenyl;
R₃ is hydrogen, alkyl of 1 to 7 carbons, phenyl, substituted phenyl, -(CH₂)_{1 to 4}-phenyl, or -(CH₂)_{1 to 4}-substituted phenyl;
R₁ is hydroxy, alkoxy of 1 to 7 carbons, or NH₂; and
n is an integer from 1 to 15.

3. The method of Claim 2 wherein:
R₂ is benzyl;
R₃ is hydrogen;
n is an integer from 1 to 9; and
R₁ is hydroxy.

4. The use of Claim 2 wherein:
R₂ is benzyl;
R₃ is hydrogen;
n is one; and
R₁ is hydroxy.

5. The use of Claim 1 wherein said endopeptidase inhibitors is a dual acting inhibitor of the formula 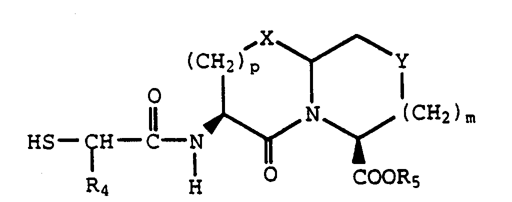 or a pharmaceutically acceptable salt thereof wherein:
p is one or two;
X is O or S;
m is zero or one;
Y is CH₂, S or O provided that Y is S or O only when m is one;
R₄ is hydrogen, alkyl of 1 to 7 carbons, phenyl, substituted phenyl, -(CH₂)_{1 to 4}-phenyl, -(CH₂)_{1 to 4}-substituted phenyl, cycloalkyl of 3 to 7 carbons, -(CH₂)_{1 to 4}-cycloalkyl of 3 to 7 carbons, heteroaryl, and -(CH₂)_{1 to 4}-heteroaryl; and
R₅ is hydrogen, alkyl of 1 to 7 carbons, -(CH₂)_{1 to 4}-phenyl and -(CH₂)_{1 to 4}-substituted phenyl.

6. The use of Claim 5 wherein:
R₄ is benzyl, cyclopropylmethyl, or straight or branched chain alkyl of 3 to 5 carbons;
p is one or two;
X is O or S;
m is zero or one;
Y is CH₂, S, or O provided that Y is S or O when m is one; and
R₅ is hydrogen.

7. The use of Claim 5 wherein:
R₄ is benzyl;
p is two;
Y is S;
m is one;
Y is CH₂ ; and
R₅ is hydrogen.

8. The use of Claim 1 wherein said endopeptidase inhibitor is a dual acting inhibitor of the formula 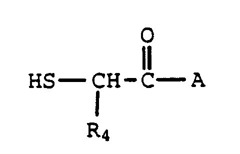 or a pharmaceutically acceptable salt thereof wherein:
A is 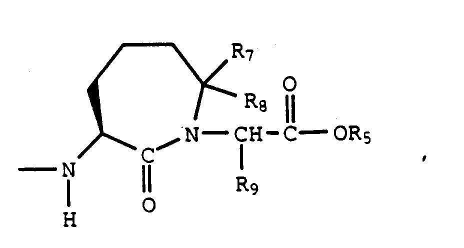 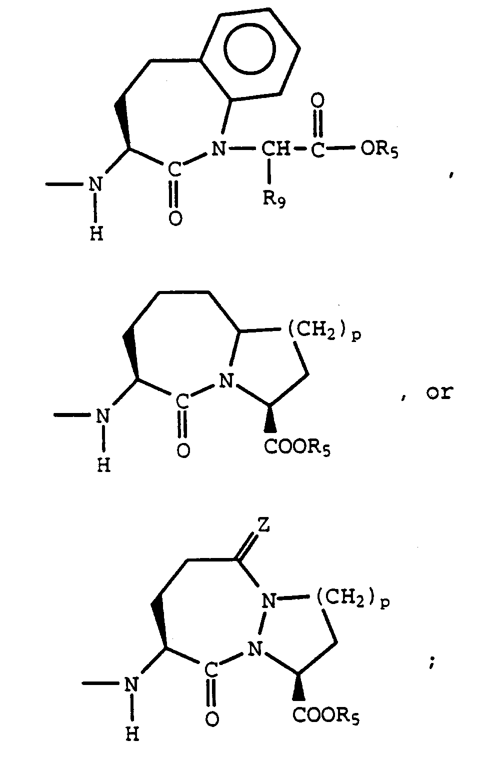 p is one or two;
R₄ is hydrogen, alkyl of 1 to 7 carbons, phenyl, substituted phenyl, -(CH₂)_{1 to 4}-phenyl, -(CH₂)_{1 to 4}-substituted phenyl, cycloalkyl of 3 to 7 carbons, -(CH₂)_{1 to 4}-cycloalkyl of 3 to 7 carbons, heteroaryl, and -(CH₂)_{1 to 4}-heteroaryl;
R₅ is hydrogen, alkyl of 1 to 7 carbons, -(CH₂)_{1 to 4}-phenyl and -(CH₂)_{1 to 4}-substituted phenyl;
R₇ and R₈ are both hydrogen, or both alkyl of 1 to 7 carbons, or R₇ is hydrogen and R₈ is alkyl of 1 to 7 carbons, phenyl, -(CH₂)_{1 to 4}-phenyl and -(CH₂)_{1 to 4}-substituted phenyl, or R₇ and R₈ taken together with the carbon to which they are attached complete a cycloalkyl of 3 to 5 carbons;
R₉ is hydrogen or alkyl of 1 to 7 carbons; and
Z is oxo or two hydrogens.

9. The use of Claim 8 wherein:
R₄ is benzyl;
R₇ and R₈ are both methyl;
R₉ is hydrogen or methyl, especially hydrogen;
p is one or two; and
Z is oxo.

10. The use of Claim 1 wherein said angiotensin II antagonist and said selective neutral endopeptidase inhibitor or said dual acting neutral endopeptidase inhibitor are administered from a single dosage form containing both types of compounds.

11. The use of Claim 1 wherein said angiotensin II antagonist and said selective neutral endopeptidase inhibitor or said dual acting neutral endopeptidase inhibitor are administered from separate dosage forms at about the same time.

12. The use of Claim 1 wherein said angiotensin II antagonist and said selective neutral endopeptidase inhibitor or said dual acting neutral endopeptidase inhibitor are administered from separate dosage forms at from within several minutes of each other up to about 4 hours apart.

13. A composition useful for treating congestive heart failure and/or hypertension comprising a pharmaceutically acceptable carrier and an effective amount of the angiotensin II antagonist 2-butyl-6,7,8,9-tetrahydro-3-[[2'-(1H-tetrazol-5-yl)-[1,1'-biphenyl]-4-yl]methyl]-1,3-diazaspiro[4.4]nonan-4-one or a pharmaceutically acceptable salt thereof and an effective amount of the selective neutral endopeptidase inhibitor of the formula 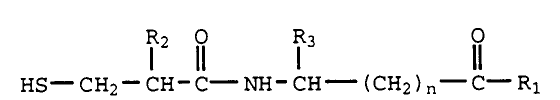 or a pharmaceutically acceptable salt thereof wherein R₁, R₂, R₃ and n are as defined in Claim 2.

14. A composition useful for treating congestive heart failure and/or hypertension comprising a pharmaceutically acceptable carrier and an effective amount of the angiotensin II antagonist 2-butyl-6,7,8,9-tetrahydro-3-[[2'-(1H-tetrazol-5-yl)-[1,1'-biphenyl]-4-yl]methyl]-1,3-diazaspiro[4.4]nonan-4-one or a pharmaceutically acceptable salt thereof and an effective amount of the dual acting neutral endopeptidase inhibitor of the formula 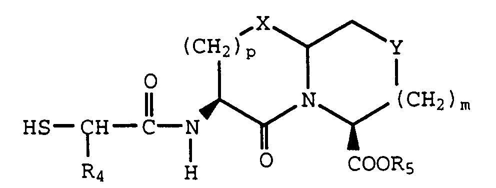 or a pharmaceutically acceptable salt thereof wherein X,Y, m, p, R₄, and R₅ are as defined in Claim 5.

15. A composition useful for treating congestive heart failure and/or hypertension comprising a pharmaceutically acceptable carrier and an effective amount of the angiotensin II antagonist 2-butyl-6,7,8,9-tetrahydro-3-[(2'-(1H-tetrazol-5-yl)-[1,1'biphenyl]-4-yl]methyl]-1,3-diazaspiro[4.4]nonan-4-one or a pharmaceutically acceptable salt thereof and an effective amount of the dual acting neutral endopeptidase inhibitor of the formula 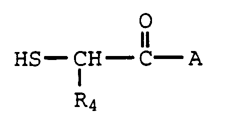 or a pharmaceutically acceptable salt thereof wherein A and R₄ are as defined in Claim 8.
